# EUROPEAN PATENT APPLICATION

(11) **EP 4 464 331 A1**
(43) Date of publication of application: **20.11.2024**
(21) Application number: 23737187.7
(22) Date of filing: 09.01.2023
(51) Int. Cl.: A61K 38/22, A61K 9/08, A61K 9/00, A61P 3/10, A61P 3/04, A61P 5/50

(54) **STABLE PHARMACEUTICAL COMPOSITION OF RECEPTOR AGONIST, AND PREPARATION METHOD AND APPLICATION THEREOF**

(30) Priority: 10.01.2022 CN 202210021736
(71) Applicant: Jiangsu Hansoh Pharmaceutical Group Co., Ltd., Lianyungang, Jiangsu 222047 (CN)
(72) Inventor: DONG, Li, Lianyungang, Jiangsu 222047 (CN); HAN, Yan, Lianyungang, Jiangsu 222047 (CN); HAO, Haohua, Lianyungang, Jiangsu 222047 (CN); YU, Jun, Lianyungang, Jiangsu 222047 (CN); SONG, Yang, Lianyungang, Jiangsu 222047 (CN)
(74) Representative: Cabinet Beau de Loménie
(86) International application number: PCT/CN2023/071277
(87) International publication number: WO 2023/131325

(57) **Abstract**

The present invention relates to a stable pharmaceutical composition of a receptor agonist, a preparation method therefor and the use thereof. In particular, the present invention discloses a pharmaceutical composition having a dual-receptor agonist as an active ingredient. The pharmaceutical composition comprises a dual-receptor agonist buffer solution, an osmotic pressure regulator and a pH regulator. The pharmaceutical composition of the present invention has good drug stability and safety, and the preparation method is simple and convenient, and suitable for industrial production.

## Description

### Technical Field

The present invention relates to the field of pharmaceutical preparations. Specifically, the present invention relates to a stable pharmaceutical composition of a GLP-1/GIP receptor agonist, a preparation method therefor and the use thereof.

### Background Art

Over the past 50 years, the prevalence of diabetes mellitus has continued to increase worldwide, and the number of people affected is expected to 700 million by 2050. The prevalence of diabetes mellitus in China is 11.2%, with about 130 million of people affected. China is currently the country with the largest number of diabetic patients, which has become one of the major public health problems, bringing huge physical, psychological and economic burdens to patients, families and society. Type 2 diabetes mellitus (T2DM) accounts for more than 90% of the diabetic population and is primarily pathophysiologically characterized by a decrease in the ability of insulin to regulate glucose metabolism (i.e., insulin resistance) with a concomitant decrease in insulin secretion caused by pancreatic β cell dysfunction.

T2DM medications fall into three main categories, oral hypoglycemic agents, GLP-1 receptor agonists (GLP-1 RAs) and insulin/insulin analogues. Oral hypoglycemic agents mainly include insulin secretagogues (sulfonylureas, non-sulfonylureas), insulin sensitizers (metformin, thiazolidinediones), etc. Metformin is the first choice for clinical treatment, which has a strong blood glucose lowering effect, a lower risk of hypoglycemia, and a body weight reducing advantage. If blood glucose control is not good, double or triple therapy is performed, in combination with insulin secretagogues, dipeptidyl peptidase-4 (DPP-4) inhibitors, GLP-1 RAs, etc. In apparently obese T2DM patients, metabolic surgery can be performed to significantly reduce body weight while alleviating or even reversing the diabetic condition. Following metabolic surgery, significant changes in intestinal hormones occur and metabolic effects such as body weight reduction and blood glucose decrease are achieved through enhanced incretin secretion effect. Incretin secretion effect is one of the main causes of increased insulin secretion after eating in healthy populations, of which the two intestinal hormones that play an important role are GLP-1 and GIP, respectively.

After eating, T2DM patients have reduced GLP-1 secretion, but after GLP-1 binds to a receptor, its effect of stimulating insulin secretion is similar to that in healthy populations. GLP-1 in humans has a very short half-life due to its rapid degradation by DPP-4. For the drug development for this mechanism, endogenous GLP-1 is engineered to function with a longer half-life in the human body. In recent decades, the development of GLP-1 RAs have progressed rapidly, and in addition to their significant blood glucose lowering effects, they also have a significant body weight reducing advantage. Compared with placebo groups, they can also significantly reduce the risk of major cardiovascular events and all-cause mortality. Besides for the treatment of diabetes mellitus, GLP-1 RAs have also been developed for the treatment of overweight or obese non-diabetic patients. Duraglutide and semaglutide in GLP1-RAs have significant effects, with duraglutide marketed in China in 2019, with significantly better blood glucose lowering and body weight reducing effects than liraglutide. The blood glucose lowering and body weight reducing effects of semaglutide are superior to those of dulaglutide, and the safety is similar to that of dulaglutide.

Long-term treatment with high concentrations of GLP-1 RAs can maintain good blood glucose lowering and body weight reducing effects, but severe gastrointestinal side effects such as nausea and vomiting limit the use of the maximum dose of GLP-1 RAs to achieve their potential maximum efficacy, so there still remains a large gap between the efficacy of GLP-1 RAs on T2DM and that of gastrointestinal metabolic surgery. At present, a multi-target drug development strategy based on the mechanism of action of GLP-1 RAs is proposed, where another GIP with incretin effect is an important research target. The blood glucose lowering effect of GIP is severely impaired at high blood glucose concentrations in T2DM patients, but its insulin secretion stimulating effect can be quickly restored when blood glucose concentrations return to normal levels. Moreover, GIP can regulate abnormal fat metabolism, and reduce abnormal fat accumulation between tissues and thus improves insulin sensitivity, while acting synergistically with GLP-1 in the central nervous system to reduce food intake. GIP can also significantly reduce gastrointestinal reactions such as nausea and vomiting caused by the antineoplastic drug cisplatin, possibly further enhancing efficacy by increasing the tolerance of GLP-1 RAs.

The active ingredient of the present invention is a new generation of GIP/GLP-1 dual-receptor agonist developed by our company, having a strong agonistic activity on both the GIP receptor and the GLP-1 receptor. With the dual-receptor agonist activity, the dual-receptor agonist is intended for the treatment of type 2 diabetes mellitus (T2DM). The pharmacodynamic experiment results in animals show that: after single administration, the present invention can significantly reduce the random blood glucose in db/db diabetic mice, stimulate insulin secretion and reduce food intake, and has a certain body weight reducing effect, and the pharmaceutical composition of the present invention has a better therapeutic effect than that of semaglutide at the same dose. After long-term continuous administration once every 3 days, the present invention can dose-dependently reduce blood glucose, body weight and liver weight in db/db diabetic mice; in the continuous administration test, the present invention can dose-dependently reduce the body weight, food intake and liver weight in DIO mice, and can also significantly reduce the triglyceride, low density lipoprotein cholesterol and total cholesterol levels in the plasma in DIO (diet-induced obesity) mice, and improve the disorder of blood lipid metabolism in DIO mice; and at the same dose, the present invention has better blood lipid lowering and body weight reducing effects than those of semaglutide. It can be seen that the active ingredient of the present invention has a good blood glucose lowering effect, can play a role in reducing blood lipid and body weight, and has a good development prospect.

The active ingredient in the present invention is a polypeptide, which is a compound formed by connecting a plurality of amino acids together via peptide bonds. The stressing test results of the bulk drug show that significant degradations occur under conditions of acids, alkalis, oxidation or high temperature; and the influencing factor test results of the bulk drug show that the bulk drug has polymer growth under conditions of high temperature or illumination. Due to the particularity of the active ingredient, which results in poor stability of the active ingredient, it remains a great challenge for researchers to obtain a stable, high-quality, industrially producible pharmaceutical composition of a GLP-1/GIP receptor agonist.

### Summary of the Invention

An objective of the present invention is to provide a stable pharmaceutical composition of a GIP/GLP-1 dual-receptor agonist.

The objective of the present invention is achieved by the following technical solutions:
the pharmaceutical composition of the present invention comprises an active ingredient, a buffer solution, an osmotic pressure regulator and a pH regulator, and the active ingredient of the present invention has a structure of:

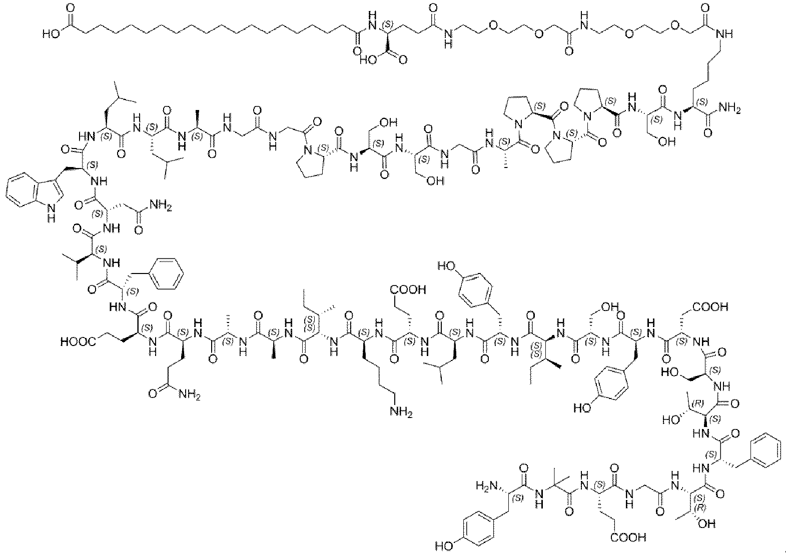

The amino acid sequence of the structure is as follows:
L-tyrosyl-isobutyryl-L-glutamyl-glycyl-L-threonyl-L-phenylalanyl-L-threonyl-L-seryl-L-aspartyl-L-tyrosyl-L-seryl-L-isoleucyl-L-tyrosyl-L-leucyl-L-glutamyl-L-lysyl-L-isoleucyl-L-alanyl-L-alanyl-L-glutaminyl-L-glutamyl-L-phenylalanyl-L-valyl-L-asparaginyl-L-tryptophanyl-L-leucyl-L-leucyl-L-alanyl-glycyl-glycyl-L-prolyl-L-seryl-L-seryl-glycyl-L-alanyl-L-prolyl-L-prolyl-L-prolyl-L-seryl-{N6-[(22S)-10,19,24-trioxo-3,6,12,15-tetraoxa-9,18,23-triaza-22,42-dicarboxy-tetradodecanoyl]}-L-lysinamide.

Preferably, the pharmaceutical composition of the GIP/GLP-1 dual-receptor agonist is an injection, and the concentration of the active ingredient is selected from 0.5 mg/mL to 40 mg/mL, preferably 1 mg/mL to 30 mg/mL, and more preferably 1 mg/mL, 2 mg/mL, 5 mg/mL, 10 mg/mL, 15 mg/mL, 20 mg/mL, 25 mg/mL or 30 mg/mL.

Preferably, the injection comprises 0.05% to 4.0% (W/V) of the active ingredient, preferably 0.1% to 4.0% (W/V), and more preferably 0.1% to 3.0% (W/V).

In the present invention, the unit of W/V is g/mL.

Preferably, the buffer solution is selected from a phosphate buffer solution, an acetate buffer solution, a citrate buffer solution, a carbonate buffer solution, a tartrate buffer solution, a Tris buffer solution and a histidine salt, preferably a citrate buffer solution or a phosphate buffer solution, and more preferably disodium hydrogen phosphate.

Preferably, the injection comprises 0.05% to 3.0% (W/V) of the buffer solution, preferably 0.05% to 2.0% (W/V), and more preferably 0.05% to 1.0% (W/V).

Preferably, the osmotic pressure regulator is selected from one or more of mannitol, lactose, sucrose, propylene glycol, and glycerol, preferably propylene glycol or mannitol.

Preferably, the injection comprises 0.05% to 5.0% (W/V) of the osmotic pressure regulator, preferably 1.0% to 3.0% (W/V), and more preferably 1.0% to 2.0% (W/V).

Preferably, the pH regulator is selected from one or more of hydrochloric acid and sodium hydroxide.

Preferably, the injection comprises 0.1% to 3.0% (W/V) of the active ingredient, 0.05% to 3.0% (W/V) of the buffer solution, 0.5% to 5.0% (W/V) of the osmotic pressure regulator, and an appropriate amount of the pH regulator.

Preferably, the injection comprises 0.1% to 3.0% (W/V) of the active ingredient, 0.05% to 2.0% (W/V) of the buffer solution, 1.0% to 3.0% (W/V) of the osmotic pressure regulator, and an appropriate amount of the pH regulator.

Preferably, the injection comprises 0.1% to 3.0% (W/V) of the active ingredient, 0.05% to 1.0% (W/V) of the buffer solution, 1.0% to 2.0% (W/V) of the osmotic pressure regulator, and an appropriate amount of the pH regulator.

Preferably, the injection comprises 0.1% to 3.0% (W/V) of the active ingredient, 0.05% to 0.1% (W/V) of the buffer solution, 1.0% to 2.0% (W/V) of the osmotic pressure regulator, and an appropriate amount of the pH regulator.

Preferably, the injection comprises 0.1% to 3.0% (W/V) of the active ingredient, 0.05% to 1.0% (W/V) of disodium hydrogen phosphate and/or sodium hydroxide, 1.0% to 2.0% (W/V) of propylene glycol, and an appropriate amount of the pH regulator.

Optionally, the injection further comprises a preservative selected from one or more of m-cresol, phenol, phenylcarbinol, phenethyl alcohol, parahydroxybenzoate ester, hydroxybenzoate, benzyl alcohol, chlorobutanol, phenoxyethanol, methyl paraben, etc., preferably m-cresol, phenol or chlorobutanol.

Optionally, the injection comprises 0.01% to 3% (W/V) of the preservative, preferably comprises 0.05% to 1.5% (W/V) of the preservative, more preferably comprises 0.1% to 1% (W/V) of the preservative, and further preferably comprises 0.1% to 0.5% (W/V) of the preservative.

Preferably, the injection comprises 0.1% to 3.0% (W/V) of the active ingredient, 0.05% to 1.0% (W/V) of disodium hydrogen phosphate and/or sodium hydroxide, 1.0% to 2.0% (W/V) of propylene glycol, 0.05% to 1.5% (W/V) of the preservative, and an appropriate amount of the pH regulator.

Preferably, the injection comprises 0.1% to 3.0% (W/V) of the active ingredient, 0.05% to 1.0% (W/V) of disodium hydrogen phosphate and/or sodium hydroxide, 1.0% to 2.0% (W/V) of propylene glycol, 0.1% to 1% (W/V) of the preservative, and an appropriate amount of pH regulator.

Preferably, the injection comprises 0.1% to 3.0% (W/V) of the active ingredient, 0.05% to 1.0% (W/V) of disodium hydrogen phosphate and/or sodium hydroxide, 1.0% to 2.0% (W/V) of propylene glycol, 0.1% to 0.5% (W/V) of the preservative, and an appropriate amount of pH regulator.

Preferably, the weight ratio of the active ingredient to the buffer solution is 1 : 0.01 to 10, preferably 1 : 0.02 to 1, more preferably 1 : 0.02 to 0.5.

Preferably, the weight ratio of the active ingredient to the osmotic pressure regulator is 1 : 0.1 to 20, preferably 1 : 0.4 to 10.

Preferably, the weight ratio of disodium hydrogen phosphate to sodium hydroxide in the buffer solution is 1 : 0 to 3.0, preferably 1 : 0.10 to 2.5, more preferably 1 : 0.13 to 1.5, and further preferably 1 : 0.13 to 1.2.

Preferably, the weight ratio of the buffer solution to the osmotic pressure regulator is 1 : 5 to 80, preferably 1 : 5 to 60, more preferably 1 : 15 to 40, and further preferably 1 : 15 to 25.

Preferably, the pH range in the pharmaceutical composition is 6.5 to 9.0, preferably 7.0 to 8.5, and more preferably 7.0 to 8.0.

Preferably, the dosage volume of the pharmaceutical composition is 0.5 ml to 1 ml.

Another objective of the present invention is to provide a method for preparing the pharmaceutical composition, which method comprises dissolving the buffer solution and the osmotic pressure regulator in water for injection, dissolving the active ingredient in the above-mentioned drug solution, stirring same for dissolution, adding the pH regulator, adjusting same to a constant volume, and filtering and subpackaging same.

Preferably, the water for injection is cooled to ≤ 25°C.

Preferably, the prepared pharmaceutical composition is subjected to aseptic filtration, subpackaging and packaging.

Preferably, the filling process is completed under nitrogen protection.

Preferably, the pharmaceutical composition is subpackaged using a prefilled syringe.

Preferably, the pharmaceutical composition is subpackaged using a cartridge bottle.

Preferably, the pharmaceutical composition is subpackaged using a vial.

Another objective of the present invention is to provide the use of the pharmaceutical composition for the manufacture of a medicament for the treatment of non-insulin-dependent diabetes mellitus, insulin-dependent diabetes mellitus, obesity, insulin resistance or disorder of blood lipid metabolism, wherein preferably, the non-insulin-dependent diabetes mellitus is type 2 diabetes mellitus.

The pharmaceutical composition of the present invention can be simultaneously, separately or successively used in combination with one or more reagents selected from metformin, thiazolidinediones, sulfonylureas, dipeptidyl peptidase inhibitors and sodium-glucose cotransporters.

Drugs, different from other chemical products, even with a very small amount of an impurity, may pose risks to the safety, efficacy, quality controllability, etc. of clinical medication. In the present invention, a pharmaceutical composition having good stability can be obtained by controlling the components and proportions of the components in the prescription without adding other auxiliary materials, where the content of related substances is effectively controlled and the content of particular impurities is significantly reduced so as to greatly reduce the toxicity of the drug, and the content of particular impurities increases slowly after the drug is placed for a long time, so as to improve the safety of the drug. Further, stability of the preparation is improved by means of filling under nitrogen protection.

### Detailed Description of Embodiments

It should be understood that a person skilled in the art can make various modifications and improvements to the present invention based on the content disclosed herein without departing from the spirit and scope of the present invention. The various modifications and improvements to the present invention should all fall within the scope of protection as defined by claims of the present application. Furthermore, it should be understood that examples provided herein are only for the purpose of illustrating the present invention and should not be construed as a limit on the present invention.

### Example 1

| Component | Amount |
|---|---|
| Active ingredient | 0.2 g, 1.0 g, 2.0 g, 3.0 g, 4.0 g |
| Disodium hydrogen phosphate¹ | 0.071 g |
| Propylene glycol | 1.5 g |
| Sodium hydroxide² | 0.01 g |
| Sodium hydroxide | Q.S. |
| Hydrochloric acid | Q.S. |
| Water for injection | Added to 100 ml |

| | |
|---|---|
| Note 1: 5 mM disodium hydrogen phosphate solution was used. Note 2: 100 g/L sodium hydroxide solution was used. | |

The water for injection was cooled to ≤ 25°C and added for later use. A prescription amount of disodium hydrogen phosphate (0.071 g) was weighed and dissolved in 90 ml of the water for injection, and stirred for dissolution; 0.1 ml of 100 g/L sodium hydroxide solution was then added and stirred uniformly (0.2 g of the active ingredient was not added); a prescription amount of propylene glycol (1.5 g) was weighed and stirred uniformly; a prescription amount of the active ingredient of the present invention was weighed and stirred slowly for dissolution, and the pH of the drug solution was adjusted to 7.5 to 8.0 with a sodium hydroxide or hydrochloric acid solution; and the water for injection was used to adjust the volume of the above-mentioned drug solution to 100 ml, finally the drug solution was filtered with a 0.22 µm microporous filtration membrane, the filtered drug solution was subpackaged into prefilled syringe bottles, the bottles were filled with nitrogen, and stoppers were pressed into the bottles. The samples were investigated for stability under conditions of 25°C ± 2°C/60% RH ± 5% RH and 40°C, respectively, and the investigation indicators are appearance and related substances. The results are as shown in the following table.

| Active ingredient | Conditions | Time | Appearance | Total impurity (%) |
|---|---|---|---|---|
| 0.2 g | | 0 d | Colorless clear liquid | 2.5 |
| | | 6 M | Colorless clear liquid | 7.0 |
| 1.0 g | | 0 d | Colorless clear liquid | 2.6 |
| | | 6 M | Colorless clear liquid | 7.3 |
| 2.0 g | 25°C ± 2°C/ 60% RH ± 5% RH | 0 d | Colorless clear liquid | 2.6 |
| | | 6 M | Colorless clear liquid | 7.3 |
| 3.0 g | | 0 d | Colorless clear liquid | 2.5 |
| | | 6 M | Colorless clear liquid | 7.2 |
| 4.0 g | | 0 d | Colorless clear liquid | 2.6 |
| | | 6 M | Colorless clear liquid | 7.3 |
| 0.2 g | | 0 d | Colorless clear liquid | 2.5 |
| | | 14 d | Colorless clear liquid | 4.6 |
| 1.0 g | | 0 d | Colorless clear liquid | 2.6 |
| | | 14 d | Colorless clear liquid | 5.0 |
| 2.0 g | 40°C | 0 d | Colorless clear liquid | 2.6 |
| | | 14 d | Colorless clear liquid | 5.0 |
| 3.0 g | | 0 d | Colorless clear liquid | 2.4 |
| | | 14 d | Colorless clear liquid | 5.1 |
| 4.0 g | | 0 d | Colorless clear liquid | 2.5 |
| | | 14 d | Colorless clear liquid | 5.3 |

### Example 2

| Component | Amount |
|---|---|
| Active ingredient | 2.0 g |
| Disodium hydrogen phosphate¹ | 0.071 g |
| Sodium hydroxide² | 0.01 g |
| Propylene glycol | 1.5 g |
| Sodium hydroxide | Q.S. |
| Hydrochloric acid | Q.S. |
| Water for injection | Added to 100 ml |

| | |
|---|---|
| Note 1: 5 mM disodium hydrogen phosphate solution was used. Note 2: 100 g/L sodium hydroxide solution was used. | |

The water for injection was cooled to ≤ 25°C and added for later use. A prescription amount of disodium hydrogen phosphate (0.071 g) was weighed and dissolved in 90 ml of the water for injection, and stirred for dissolution; 0.1 ml of 100 g/L sodium hydroxide solution was then added and stirred uniformly; a prescription amount of propylene glycol (1.5 g) was weighed and stirred uniformly; a prescription amount of the active ingredient (2.0 g) of the present invention was weighed and stirred slowly for dissolution, and the pH of the drug solution was adjusted to 6.5, 7.0, 7.5, 7.8, 8.0 and 8.5 with a sodium hydroxide or hydrochloric acid solution, respectively; and the water for injection was used to adjust the volumes of the above-mentioned drug solutions to 100 ml, respectively, finally the drug solutions were each filtered with a 0.22 µm microporous filtration membrane, the filtered drug solutions were each subpackaged into prefilled syringe bottles, the bottles were filled with nitrogen, and stoppers were pressed into the bottles. The samples were investigated for stability under conditions of 25°C ± 2°C/60% RH ± 5% RH, and the investigation indicators are appearance and related substances. The results are as shown in the following table.

| pH | Conditions | Time | Appearance | Total impurity (%) |
|---|---|---|---|---|
| 6.5 | | 0 d | Colorless clear liquid | 2.2 |
| | | 1 M | Transparent gelatinous precipitation | / |
| 7.0 | | 0 d | Colorless clear liquid | 2.2 |
| | | 1 M | Colorless clear liquid | 2.5 |
| 7.5 | | 0 d | Colorless clear liquid | 2.2 |
| | 25°C ± 2°C/ 60% RH ± 5% RH | 1 M | Colorless clear liquid | 2.9 |
| 7.8 | | 0 d | Colorless clear liquid | 2.2 |
| | | 1 M | Colorless clear liquid | 3.0 |
| 8.0 | | 0 d | Colorless clear liquid | 2.1 |
| | | 1 M | Colorless clear liquid | 3.1 |
| 8.5 | | 0 d | Colorless clear liquid | 2.1 |
| | | 1 M | Colorless clear liquid | 3.6 |

### Example 3

| Component | Amount |
|---|---|
| Active ingredient | 2.0 g |
| Disodium hydrogen phosphate¹ | 0.071 g |
| Sodium hydroxide² | Prescription amount |
| Propylene glycol | 1.5 g |
| Water for injection | Added to 90 ml |

| | |
|---|---|
| Note 1: 5 mM disodium hydrogen phosphate solution was used. Note 2: 100 g/L sodium hydroxide solution was used. | |

The water for injection was cooled to ≤ 25°C and added for later use. A prescription amount of disodium hydrogen phosphate (0.071 g) was weighed and dissolved in 90 ml of the water for injection, and stirred for dissolution; a prescription amount of 100 g/L sodium hydroxide solution was then added and stirred uniformly; a prescription amount of propylene glycol (1.5 g) was weighed and stirred uniformly; and the pH value of the solution was determined, a prescription amount of the active ingredient (2.0 g) of the present invention was weighed, and slowly added and stirred for 45 min, then the dissolution phenomenon of the active ingredient was observed and the pH value of the solution was determined, the samples were investigated for stability under conditions of 25°C ± 2°C/60% RH ± 5% RH, 5°C ± 3°C, 40°C and illumination, respectively, the impurity components of the samples were subjected to analysis study to investigate the effects of different buffer solution concentrations on the impurity components, which provide research idea for screening the pharmaceutical composition. The study results are as shown in the following table.

| Adding amount of 100 g/L sodium hydroxide | pH before adding active ingredient | pH after adding active ingredient | Dissolution phenomenon | Investigation conditions | Time | Related substances (%) | | |
|---|---|---|---|---|---|---|---|---|
| | | | | | | RRT1.0 6 | RRT1.14 | Total impurity |
| 0.8 ml | 12.0 | 6.8 | Colorless clear liquid, completely dissolved | 0 d | | 0.20 | 0.02 | 2.1 |
| | | | | 25°C ± 2°C/ 60% RH ± 5% RH | 1 M | 0.24 | 0.28 | 4.4 |
| | | | | 5°C ± 3°C | 1 M | 0.25 | 0.30 | 4.5 |
| | | | | 40°C | 1 M | 0.31 | 0.58 | 6.7 |
| | | | | Illumination | 15 d | 0.30 | 0.70 | 7.5 |
| 0.3 ml | 11.7 | 6.6 | Colorless clear liquid, completely dissolved | 0 d | | 0.18 | 0.02 | 2.0 |
| | | | | 25°C ± 2°C/ 60% RH ± 5% RH | 1 M | 0.23 | 0.25 | 4.3 |
| | | | | 5°C ± 3°C | 1 M | 0.22 | 0.35 | 4.3 |
| | | | | 40°C | 1 M | 0.29 | 0.59 | 6.6 |
| | | | | Illumination | 15 d | 0.33 | 0.65 | 7.3 |
| 0.1 ml | 10.8 | 5.7 | Colorless clear liquid, completely dissolved | 0 d | | 0.20 | Not detected | 2.0 |
| | | | | 25°C ± 2°C/ 60% RH ± 5% RH | 1 M | 0.22 | 0.25 | 4.3 |
| | | | | 5°C ± 3°C | 1 M | 0.22 | 0.35 | 4.2 |
| | | | | 40°C | 1 M | 0.28 | 0.55 | 6.5 |
| | | | | Illumination | 15 d | 0.38 | 0.65 | 7.2 |
| 0.08 ml | 10.3 | / | Off-white solution, incompletely dissolved | 0 d | | 0.23 | 0.45 | 9.0 |

### Example 4

| Component | Amount |
|---|---|
| Active ingredient | 2.0 g |
| Disodium hydrogen phosphate | Prescription amount |
| Sodium hydroxide¹ | 0.01 g |
| Propylene glycol | 1.5 g |
| Sodium hydroxide | Q.S. |
| Hydrochloric acid | Q.S. |
| Water for injection | Added to 100 ml |

| | |
|---|---|
| Note 1: 100 g/L sodium hydroxide solution was used. | |

The water for injection was cooled to ≤ 25°C and added for later use. A prescription amount of disodium hydrogen phosphate was weighed and dissolved in 90 ml of the water for injection, and stirred for dissolution; 0.1 ml of 100 g/L sodium hydroxide solution was then added and stirred uniformly; a prescription amount of propylene glycol (1.5 g) was weighed and stirred uniformly; a prescription amount of the active ingredient (2.0 g) of the present invention was weighed and stirred slowly for dissolution, and the pH of the drug solution was adjusted to 7.5 to 8.0 with a sodium hydroxide or hydrochloric acid solution, respectively; and the water for injection was used to adjust the volume of the above-mentioned drug solution to 100 ml, finally the drug solution was filtered with a 0.22 µm microporous filtration membrane, the filtered drug solution was subpackaged into prefilled injection bottles, the bottles were filled with nitrogen, and stoppers were pressed into the bottles. The samples were investigated for stability under conditions of 25°C ± 2°C/60% RH ± 5% RH or 40°C, and the investigation indicators are appearance and related substances. The results are as shown in the following table.

| Concentration of disodium hydrogen phosphate | Conditions | Time | Appearance | Total impurity (%) |
|---|---|---|---|---|
| 4 mM | | 0 d | Colorless clear liquid | 2.7 |
| | | 1 M | Colorless clear liquid | 3.8 |
| 5 mM | | 0 d | Colorless clear liquid | 2.7 |
| | | 1 M | Colorless clear liquid | 3.5 |
| 6 mM | | 0 d | Colorless clear liquid | 2.7 |
| | | 1 M | Colorless clear liquid | 3.6 |
| 7 mM | 25°C ± 2°C/ 60% RH ± 5% RH | 0 d | Colorless clear liquid | 2.0 |
| | | 1 M | Colorless clear liquid | 3.6 |
| 8 mM | | 0 d | Colorless clear liquid | 2.1 |
| | | 1 M | Colorless clear liquid | 3.6 |
| 11 mM | | 0 d | Colorless clear liquid | 2.1 |
| | | 1 M | Colorless clear liquid | 3.7 |
| 4 mM | | 0 d | Colorless clear liquid | 2.7 |
| | | 14 d | Colorless clear liquid | 4.1 |
| 5 mM | | 0 d | Colorless clear liquid | 2.7 |
| | | 14 d | Colorless clear liquid | 3.8 |
| 6 mM | | 0 d | Colorless clear liquid | 2.7 |
| | 40°C | 14 d | Colorless clear liquid | 4.1 |
| 7 mM | | 0 d | Colorless clear liquid | 2.0 |
| | | 1 M | Colorless clear liquid | 4.4 |
| 8 mM | | 0 d | Colorless clear liquid | 2.1 |
| | | 1 M | Colorless clear liquid | 4.3 |
| 11 mM | | 0 d | Colorless clear liquid | 2.1 |
| | | 14 d | Colorless clear liquid | 4.5 |

### Example 5

| Component | Amount |
|---|---|
| Active ingredient | 2.0 g |
| Disodium hydrogen phosphate¹ | 0.071 g |
| Sodium hydroxide² | 0.01 g |
| Osmotic pressure regulator | Prescription amount |
| Sodium hydroxide | Q.S. |
| Hydrochloric acid | Q.S. |
| Water for injection | Added to 100 ml |

| | |
|---|---|
| Note 1: 5 mM disodium hydrogen phosphate solution was used. Note 2: 100 g/L sodium hydroxide solution was used. | |

The water for injection was cooled to ≤ 25°C and added for later use. A prescription amount of disodium hydrogen phosphate (0.071 g) was weighed and dissolved in 90 ml of the water for injection, and stirred for dissolution; 0.1 ml of 100 g/L sodium hydroxide solution was then added and stirred uniformly; a prescription amount of the osmotic pressure regulator was weighed and stirred uniformly; a prescription amount of the active ingredient (2.0 g) of the present invention was weighed and stirred slowly for dissolution, and the pH of the drug solution was adjusted to 7.5 to 8.0 with a sodium hydroxide or hydrochloric acid solution, respectively; and the water for injection was used to adjust the volume of the above-mentioned drug solution to 100 ml, finally the drug solution was filtered with a 0.22 µm microporous filtration membrane, the filtered drug solution was subpackaged into prefilled injection bottles, the bottles were filled with nitrogen, and stoppers were pressed into the bottles. The samples were investigated for stability under conditions of 25°C ± 2°C/60% RH ± 5% RH, and the investigation indicators are appearance and related substances. The results are as shown in the following table.

| Osmotic pressure regulator | Conditions | Time | Appearance | Total impurity (%) |
|---|---|---|---|---|
| Propylene glycol 1.5 g | | 0 d | Colorless clear liquid | 2.6 |
| | | 1 M | Colorless clear liquid | 3.4 |
| Mannitol 4.64 g | | 0 d | Colorless clear liquid | 2.7 |
| | | 1 M | Colorless clear liquid | 3.7 |
| Glycerol 3.6 g | 25°C ± 2°C/ 60% RH ± 5% RH | 0 d | Colorless clear liquid | 4.2 |
| | | 1 M | Colorless clear liquid | 5.8 |
| Lactose 9.5 g | | 0 d | Colorless clear liquid | 4.4 |
| | | 1 M | Colorless clear liquid | 5.6 |
| Sucrose 9.0 g | | 0 d | Colorless clear liquid | 4.2 |
| | | 1 M | Colorless clear liquid | 5.8 |

### Example 6

| Component | Amount |
|---|---|
| Active ingredient | 2.0 g |
| Disodium hydrogen phosphate | Prescription amount |
| Sodium hydroxide¹ | 0.01 g |
| Propylene glycol | Prescription amount |
| Sodium hydroxide | Q.S. |
| Hydrochloric acid | Q.S. |
| Water for injection | Added to 100 ml |

| | |
|---|---|
| Note 1: 100 g/L sodium hydroxide solution was used. | |

The water for injection was cooled to ≤ 25°C and added for later use. A prescription amount of disodium hydrogen phosphate was weighed and dissolved in 90 ml of the water for injection, and stirred for dissolution; 0.1 ml of 100 g/L sodium hydroxide solution was then added and stirred uniformly; a prescription amount of propylene glycol was weighed and stirred uniformly; a prescription amount of the active ingredient (2.0 g) of the present invention was weighed and stirred slowly for dissolution, and the pH of the drug solution was adjusted to 7.5 to 8.0 with a sodium hydroxide or hydrochloric acid solution, respectively; and the water for injection was used to adjust the volume of the above-mentioned drug solution to 100 ml, finally the drug solution was filtered with a 0.22 µm microporous filtration membrane, the filtered drug solution was subpackaged into prefilled injection bottles, and stoppers were pressed into the bottles. The samples were investigated for stability under conditions of 25°C ± 2°C/60% RH ± 5% RH, 5°C ± 3°C, 40°C, illumination and low temperature cycling, respectively, and the investigation indicators are appearance and related substances. The results are as shown in the following table.

| Disodium hydrogen phosphate: propylene glycol (W/V%) | Conditions | | Appearance | Total impurity (%) |
|---|---|---|---|---|
| 1 : 15 | 0 d | | Colorless clear liquid | 2.1 |
| | 25°C ± 2°C/60% RH ± 5% RH | 1 M | Colorless clear liquid | 4.4 |
| | 5°C ± 3°C | 1 M | Colorless clear liquid | 4.2 |
| | 40°C | 7 d | Colorless clear liquid | 4.5 |
| | Illumination | 15 d | Colorless clear liquid | 5.4 |
| | Low temperature cycling | 2 times | Colorless clear liquid | 4.3 |
| 1 : 20 | 0 d | | Colorless clear liquid | 2.0 |
| | 25°C ± 2°C/60% RH ± 5% RH | 1 M | Colorless clear liquid | 4.3 |
| | 5°C ± 3°C | 1 M | Colorless clear liquid | 4.2 |
| | 40°C | 7 d | Colorless clear liquid | 4.7 |
| | Illumination | 15 d | Colorless clear liquid | 5.5 |
| | Low temperature cycling | 3 times | Colorless clear liquid | 4.5 |
| 1 : 25 | 0 d | | Colorless clear liquid | 2.0 |
| | 25°C ± 2°C/60% RH ± 5% RH | 1 M | Colorless clear liquid | 4.0 |
| | 5°C ± 3°C | 1 M | Colorless clear liquid | 4.2 |
| | 40°C | 7 d | Colorless clear liquid | 4.8 |
| | Illumination | 15 d | Colorless clear liquid | 5.6 |
| | Low temperature cycling | 3 times | Colorless clear liquid | 4.6 |
| 1: 40 | 0 d | | Colorless clear liquid | 2.0 |
| | 25°C ± 2°C/60% RH ± 5% RH | 1 M | Colorless clear liquid | 4.0 |
| | 5°C + 3°C | 1 M | Colorless clear liquid | 4.4 |
| | 40°C | 7 d | Colorless clear liquid | 4.7 |
| | Illumination | 15 d | Colorless clear liquid | 5.5 |
| | Low temperature cycling | 3 times | Colorless clear liquid | 4.7 |
| 1: 80 | 0 d | | Colorless clear liquid | |
| | 25°C ± 2°C/60% RH + 5% RH | 1 M | White gelatinous precipitation | Not detected |
| | 5°C ± 3°C | 1 M | White gelatinous precipitation | Not detected |
| | 40°C | 7 d | Colorless clear liquid | 5.9 |
| | Illumination | 15 d | Colorless clear liquid | 7.0 |
| | Low temperature cycling | 3 times | Colorless clear liquid | 5.8 |

### Test example 1 Hemolysis test

### 1. Test compound:

test sample: the injection (active ingredient 2.0 g) prepared in example 1;
1.2 Vehicle: sodium chloride injection.

### 2. Experimental method:

Different volumes (0.5 to 0.1 mL) of the test samples and different volumes (2.0 to 2.4 mL) of sodium chloride injections were respectively added into glass test tubes into which 2.5 mL of 2% rabbit erythrocyte suspension was added, and the sodium chloride injection and sterilized water for injection were used as a negative control and a positive control, respectively, with a total volume of 5.0 mL per test tube. Each test tube was incubated in an electric thermostat (the temperature was set as 37°C) for 3 hours, and the lysis and aggregation of erythrocytes were observed.

In the test tube containing 0.5 to 0.1 mL of the test sample and the negative control, it could be seen that the erythrocytes were deposited at the bottom of the tube, the upper solution was colorless and clear, and after shaking, the erythrocytes at the bottom of the tube were uniformly dispersed, with no hemolysis and no aggregation; and in the positive control tube containing sterile water for injection, it could be seen that some erythrocytes were deposited at the bottom of the tube, the upper solution was clear and red, and after shaking, the erythrocytes at the bottom of the tube were uniformly dispersed, with partial hemolysis and no aggregation.

Under the experimental conditions, in the experimental system, 0.1 to 0.5 mL of the injection of the pharmaceutical composition of the present invention at a labeled concentration of 20 mg/mL did not exhibit rabbit erythrocyte hemolysis, and did not cause erythrocyte aggregation.

### Test Example 2 Subcutaneous irritation study

### 1. Test compound:

test sample: the injection (active ingredient 2.0 g) prepared in example 1;

### 2. Experimental method:

New Zealand rabbits were selected for the experiment, the test sample was subcutaneously administrated to the left and right sides of the same Zealand rabbit for self-control, and the local subcutaneous irritation response at the administration sites produced by a total of 4 subcutaneous injections was observed. During the experiment, no abnormality related to the injection of the pharmaceutical composition of the present invention was found in the local administration sites in all animals.

Test Example 3 Study on therapeutic effect in type 2 diabetic db/db mice after single subcutaneous injection of the pharmaceutical composition of the present application

### 1. Test compound:

test sample: the injection (active ingredient 2.0 g) prepared in example 1;

### 2. Experimental method

The effects on blood glucose, serum insulin level, body weight and food intake in the type 2 diabetic db/db mice after the single administration were investigated while observing the duration of efficacy after the single administration. The experimental results show that the pharmaceutical composition of the present invention can significantly lower random blood glucose in the db/db mice after a single subcutaneous injection at a dose of 1, 3 or 10 nmol/kg, and shows a good dose-response relationship, while at a dose of 3 nmol/kg, it shows a significant random blood glucose lowering effect. The duration of the blood glucose lowering effect of the pharmaceutical composition of the present invention after the single subcutaneous injection was dose-related, with the duration of the random blood glucose lowering effect at a dose of 1 nmol/kg being 1 day, and the duration of the random blood glucose lowering effect at doses of 3 nmol/kg and 10 nmol/kg being 2-3 days.

**Effects of the pharmaceutical composition of the present invention on random blood glucose decrease rate in db/db mice after single administration**

| Groups | Vehicle | Pharmaceutical composition of the present invention | | |
|---|---|---|---|---|
| Dose (nmol/kg) | - | 1 | 3 | 10 |
| Time | | Blood glucose decrease rate (Mean) | | |
| Before administration | - | - | - | - |
| 1 h | 5.30 | 12.27 | 35.84*** | 38.65*** |
| 2 h | -12.99 | 6.57 | 27.78** | 39.07*** |
| 4 h | 5.53 | 10.00 | 27.24 | 43.05*** |
| 6 h | -12.54 | 23.30* | 33.84** | 43.32*** |
| 8 h | 10.19 | 26.40 | 28.30 | 51.93*** |
| 24 h | -13.98 | -2.62 | 19.21** | 26.70*** |
| 32 h | -18.47 | -8.84 | 21.55*** | 36.97*** |
| 48 h | 4.73 | 1.89 | 12.84 | 23.67 |
| 72 h | 10.57 | -7.02 | 2.00 | 9.05 |
| 96 h | 4.96 | -14.42 | 4.92 | -6.30 |
| 120 h | -0.19 | -22.01 | 9.46 | 1.25 |
| 144 h | -18.18 | -38.41 | -3.38 | 2.58 |

| | | | | |
|---|---|---|---|---|
| A difference in blood glucose decrease rate between groups was analyzed using Dunnett's multiple comparisons test in Two-way ANOVA. Compared with vehicle group, **p* < 0.05, ***p* < 0.01, ****p* < 0.001 | | | | |

In addition, Mouse Insulin ELISA Kit was used to determine the serum insulin levels in db/db mice in each group at 2 h after administration. The results show that after the single subcutaneous injection of the pharmaceutical composition of the present invention in db/db mice at 1, 3, and 10 nmol/kg, the serum insulin levels in the mice in each dose group were significantly increased compared with those in the vehicle group, and it shows a good dose-effect relationship; in addition, an increase in serum insulin is also positively correlated with a decrease in blood glucose.

**Effects of the pharmaceutical composition of the present invention on serum insulin in db/db mice at 2 hours after single administration 4 times, respectively**

| Groups | Number of animals | Insulin (µIU/mL) | Increase rate (%) |
|---|---|---|---|
| Vehicle group | 16 | 1,838.86 ± 359.90 | 0.00 |
| Pharmaceutical composition of the present invention, 1 nmol/kg | 16 | 5,170.54 ± 1,103.22*^{.} | 181.18 |
| Pharmaceutical composition of the present invention, 3 nmol/kg | 16 | 7,131.29 ± 1,178.14** | 287.81 |
| Pharmaceutical composition of the present invention, 10 nmol/kg | 16 | 8,093.32 ± 1,490.42*** | 340.13 |

A difference in serum insulin between groups was analyzed using Fisher's LSD test in One-way ANOVA. Compared with vehicle group, **p* < 0.05, ***p* < 0.01, ****p* < 0.001

Test Example 4 Study on therapeutic effect in type 2 diabetic db/db mice after long-term subcutaneous injection of the pharmaceutical composition of the present application

### 1. Test compound:

test sample: the injection (active ingredient 2.0 g) prepared in example 1;

### 2. Experimental method:

Type 2 diabetic db/db mice were administered once every 3 days for 9 consecutive times, and after the end of the experiment (d 26), the liver weights of the db/db mice in the 1, 3, and 10 nmol/kg groups were 2.313 ± 0.113 g, 2.267 ± 0.108 g, and 1.993 ± 0.104 g, respectively, all of which were significantly decreased compared with those in the vehicle group (p < 0.05, p < 0.05, p < 0.001); and there was no statistical difference in pancreas weight compared with the vehicle group (p > 0.05).

In conclusion, the pharmaceutical composition can dose-dependently reduce body weights and liver weights in type 2 diabetic db/db mice by long-term continuous administration, once every 3 days; and the random blood glucose lowering effect is obvious at doses of 3 and 10 nmol/kg.

Test Example 5 Pharmacokinetic Study in cynomolgus monkeys after single subcutaneous or intravenous injection of the pharmaceutical composition of the present application

### 1. Test compound:

the injection prepared in example 3;

### 2. Experimental method:

The administration doses for 4 groups, i.e., intravenous injection group, low-dose subcutaneous injection group, medium-dose subcutaneous group and high-dose subcutaneous injection group, were 0.1, 0.03, 0.1, and 0.3 mg/kg, respectively, all by single administration.

After the intravenous administration, the drug of the present invention had an elimination half-life t1/2 between 50.7 and 51.5 h in cynomolgus monkeys, indicating slow elimination; the drug of the present invention had a clearance CL between 0.89 and 0.93 mL/h/kg, indicating low clearance; and the drug of the present invention had an apparent volume of distribution Vd between 66.0 and 68.1 mL/kg, indicating that the drug was mainly distributed in plasma. After the single subcutaneous injection, the drug of the present invention had a time to peak drug concentration Tmax of 13.3-18.0 h in cynomolgus monkeys; the drug of the present invention had an elimination half-life T1/2 between 48.9 and 65.4 h, indicating slow elimination; and there was no statistical difference (P > 0.05) in the exposure (AUCₗₐₛₜ) of the drug of the present invention in animals of different sexes. In the dose range of 0.02 to 0.2 mg/kg, the exposure (AUCₗₐₛₜ) of the pharmaceutical composition in the plasma of the animals was increased with the increase of the dose, and the proportion of the exposure increase was close to that of the dose increase.

## Claims

1. A stable pharmaceutical composition of a GIP/GLP-1 dual-receptor agonist, wherein the stable pharmaceutical composition comprises an active ingredient, a buffer solution, an osmotic pressure regulator and a pH regulator, and the active ingredient has a structure of:

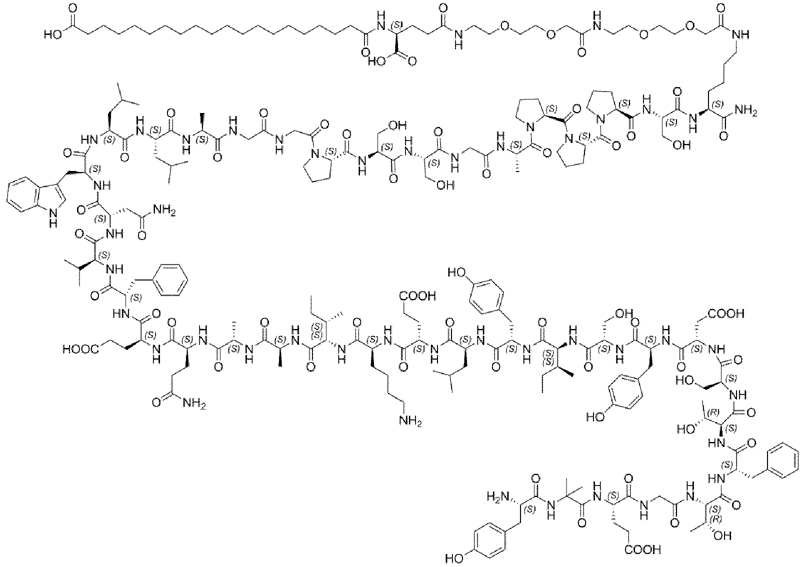

2. The pharmaceutical composition according to claim 1, wherein the pharmaceutical composition is an injection, and the concentration of the active ingredient is selected from 0.5 mg/mL to 40 mg/mL, preferably 1 mg/mL to 30 mg/mL.

3. The pharmaceutical composition according to claim 1, wherein the pharmaceutical composition comprises 0.05% to 4.0% (W/V) of the active ingredient, preferably 0.1% to 3.0% (W/V).

4. The pharmaceutical composition according to claim 1, wherein the buffer solution is selected from a phosphate buffer solution, an acetate buffer solution, a citrate buffer solution, a carbonate buffer solution, a tartrate buffer solution, a Tris buffer solution and a histidine salt, preferably a citrate buffer solution or a phosphate buffer solution, and more preferably disodium hydrogen phosphate.

5. The pharmaceutical composition according to claim 1, wherein the pharmaceutical composition comprises 0.05% to 3.0% (W/V) of the buffer solution, preferably 0.05% to 2.0% (W/V), and more preferably 0.05% to 1.0% (W/V).

6. The pharmaceutical composition according to claim 1, wherein the osmotic pressure regulator is selected from one or more of mannitol, lactose, sucrose, propylene glycol and glycerol, preferably propylene glycol or mannitol.

7. The pharmaceutical composition according to claim 1, wherein the injection comprises 0.05% to 5.0% (W/V) of the osmotic pressure regulator, preferably 1.0% to 3.0% (W/V), and more preferably 1.0% to 2.0% (W/V).

8. The pharmaceutical composition according to claim 1, wherein the pH regulator is selected from one or more of hydrochloric acid and sodium hydroxide.

9. The pharmaceutical composition according to claim 1, wherein the pharmaceutical composition comprises 0.1% to 3.0% (W/V) of the active ingredient, 0.05% to 3.0% (W/V) of the buffer solution, 0.5% to 5.0% (W/V) of the osmotic pressure regulator, and an appropriate amount of the pH regulator;
preferably the pharmaceutical composition comprises 0.1% to 3.0% (W/V) of the active ingredient, 0.05% to 2.0% (W/V) of the buffer solution, 1.0% to 3.0% (W/V) of the osmotic pressure regulator, and an appropriate amount of the pH regulator;
more preferably the pharmaceutical composition comprises 0.1% to 3.0% (W/V) of the active ingredient, 0.05% to 1.0% (W/V) of the buffer solution, 1.0% to 2.0% (W/V) of the osmotic pressure regulator, and an appropriate amount of the pH regulator; and
further preferably, the pharmaceutical composition comprises 0.1% to 3.0% (W/V) of the active ingredient, 0.05% to 1.0% (W/V) of disodium hydrogen phosphate and/or sodium hydroxide, 1.0% to 2.0% (W/V) of propylene glycol, and an appropriate amount of the pH regulator.

10. The pharmaceutical composition according to claim 1, wherein the weight ratio of the active ingredient to the buffer solution is 1 : 0.01 to 10, preferably 1 : 0.02 to 1, and more preferably 1 : 0.02 to 0.5.

11. The pharmaceutical composition according to claim 1, wherein the weight ratio of the active ingredient to the osmotic pressure regulator is 1 : 0.1 to 20, preferably 1: 0.4 to 10.

12. The pharmaceutical composition according to claim 9, wherein the buffer solution comprises disodium hydrogen phosphate and sodium hydroxide, and the weight ratio of disodium hydrogen phosphate to sodium hydroxide is 1 : 0 to 3.0, preferably 1: 0.10 to 2.5, more preferably 1: 0.13 to 1.5, and further preferably 1: 0.13 to 1.2.

13. The pharmaceutical composition according to claim 1, wherein the weight ratio of the buffer solution to the osmotic pressure regulator is 1 : 5 to 80, preferably 1 : 5 to 60, more preferably 1 : 15 to 40, and further preferably 1 : 15 to 25.

14. The pharmaceutical composition according to claim 1, wherein the pH range in the pharmaceutical composition is 6.5 to 9.0, preferably 7.0 to 8.5, and more preferably 7.0 to 8.0.

15. The pharmaceutical composition according to claims 1 to 14, wherein the pharmaceutical composition further comprises a preservative selected from one or more of m-cresol, phenol, phenylcarbinol, phenethyl alcohol, parahydroxybenzoate ester, hydroxybenzoate, benzyl alcohol, chlorobutanol, phenoxyethanol and methyl paraben.

16. A method for preparing the pharmaceutical composition according to claims 1 to 15, wherein the method comprises dissolving the buffer solution and the osmotic pressure regulator in water for injection, dissolving the active ingredient in the above-mentioned drug solution, stirring same for dissolution, adding the pH regulator, adjusting same to a constant volume, and filtering and subpackaging same.

17. Use of the pharmaceutical composition according to claims 1 to 15 for the manufacture of a medicament for the treatment of non-insulin-dependent diabetes mellitus, insulin-dependent diabetes mellitus, obesity, insulin resistance or disorder of blood lipid metabolism, wherein preferably, the non-insulin-dependent diabetes mellitus is type 2 diabetes mellitus.
